(19) Europäisches Patentamt

European Patent Office

Office européen des brevets

(11) Numéro de publication: **0 298 867 B1**

(12) **FASCICULE DE BREVET EUROPEEN**

(45) Date de publication de fascicule du brevet: **23.09.92** (51) Int. Cl.⁵: **C07C 219/08**

(21) Numéro de dépôt: **88401771.6**

(22) Date de dépôt: **07.07.88**

(54) **Procédé de fabrication de (meth)acrylate de dialkylaminoalkyle.**

(30) Priorité: **08.07.87 FR 8709697**

(43) Date de publication de la demande:
**11.01.89 Bulletin 89/02**

(45) Mention de la délivrance du brevet:
**23.09.92 Bulletin 92/39**

(84) Etats contractants désignés:
**AT BE CH DE ES GB GR IT LI LU NL SE**

(56) Documents cités:
**EP-A- 0 118 639**
**DE-A- 1 543 279**
**FR-A- 1 568 382**

(73) Titulaire: **ELF ATOCHEM S.A.**
**4 & 8, Cours Michelet La Défense 10**
**F-92800 Puteaux(FR)**

(72) Inventeur: **Hurtel, Patrice**
**2 Chemin des Brasseurs Résidence Foch**
**F-57500 Saint Avold(FR)**
Inventeur: **Hazan, Charles**
**15, Passage Trubert Bellier**
**F-75013 Paris(FR)**
Inventeur: **De Champs, François**
**6, rue Gabriel Pierné**
**F-57500 Saint Avold(FR)**
Inventeur: **Paul, Jean-Michel**
**143, route d'Espagne**
**F-31057 Toulouse(FR)**

(74) Mandataire: **Rieux, Michel et al**
**ATOCHEM Département Propriété Industriel-**
**le 4, Cours Michelet - La Défense 10 - Cedex**
**42**
**F-92091 Paris-La Défense(FR)**

## Description

La présente invention a pour objet un procédé de fabrication de (méth)acrylates de dialkylaminoalkyle (I) de formule

$$H_2C = C(R_1)-C(O)-O-A-N(R_2)(R_3)$$

dans laquelle :
- R₁ est un atome d'hydrogène ou un radical méthyle,
- A est un radical alkyle, linéaire ou ramifié, comportant 1 à 5 atomes de carbone,
- R₂ et R₃, identiques ou différents l'un de l'autre, sont un radical alkyle contenant 1 à 5 atomes de carbone,

On connaît différents procédés de fabrication des esters (méth)acryliques.

Selon le brevet britannique 960 005, les esters (méth)acryliques de formule $H_2C = CX\text{-}COOR$, R étant un radical alkyle ayant au moins 3 atomes de carbone et X étant un atome d'hydrogène ou un radical méthyle, sont préparés en faisant réagir un ester $H_2C = CX\text{-}COOR'$ dans lequel R' est un radical alkyle ayant au plus 3 atomes de carbone et un alcool ROH, R ayant la signification précise ci-dessus, en présence de titanate d'alkyle Ti(OR")₄ telque le titanate de tétraéthyle. Le principal défaut de ce procédé résulte de l'apparition d'impuretés, notamment de l'ester $H_2C = CX\text{-}COOR"$ et l'alcool R"OH issus de la transestérification des titanates d'alkyles soit avec l'alcool léger R'OH libéré au cours de la réaction, soit avec l'alcool supérieur ROH.

Selon le brevet français 1544542, les esters acryliques tels que l'acrylate de diméthylaminoéthyle sont préparés à partir d'esters acryliques de formule $H_2C = CHCOO(CH_2)n\text{-}CH(CH_3)_2$ dans laquelle n est égal à 0 ou 1, et d'alcools, en présence de catalyseur tel que les titanates de n- et isopropyle, les titanates d'isobutyle, le polybutyltitanate.

On retrouve à nouveau dans ce procédé les problèmes dus à la transestérification du catalyseur qui provoque l'apparition d'impuretés dans le mélange réactionnel, et complique la purification de l'ester acrylique préparé.

La présente invention a pour objectif de résoudre les inconvénients des procédés précités, c'est-à-dire la fabrication de (méth)acrylates de dialkylaminoalkyle (I) de grande pureté exempts de traces de (méth)-acrylates ou d'alcools à point d'ébullition voisin de celui du (méth)acrylate préparé.

Plus précisément, la présente invention a pour objet un procédé de fabrication de (méth)acrylates de dialkylaminoalkyle (I) de formule :

$$H_2C = C(R_1)-C(O)-O-A-N(R_2)(R_3)$$

dans laquelle :
- R₁ est un atome d'hydrogène ou un radical méthyle,
- A est un radical alkyle, linéaire ou ramifié, comportant 1 à 5 atomes de carbone,
- R₂ et R₃, identiques ou différents l'un de l'autre, sont un radical alkyle contenant 1 à 5 atomes de carbone,

procédé caractérisé en ce que l'on fait réagir, en présence d'au moins un inhibiteur de polymérisation, à une température comprise entre 20 et 120° C et à une pression égale ou inférieure à la pression atmosphérique, le (méth)acrylate d'éthyle avec un aminoalcool (II) de formule $(R_3)(R_2)N\text{-}A\text{-}OH$ dans laquelle A est un radical alkyle, linéaire ou ramifié, comportant 1 à 5 atomes de carbone, dans un rapport molaire (méth)acrylate d'éthyle sur aminoalcool (II) compris entre 1,5 et 5, en présence de titanate de tétraéthyle, en ce que, pendant la réaction, on soutire le mélange azéotropique (méth)acrylate d'éthyle-éthanol, et en ce que, en fin de réaction, on sépare le (méth)acrylate de dialkylaminoalkyle (I) obtenu.

La séparation du (méth)acrylate de dialkylaminoalkyle est généralement effectuée par distillation.

Par (méth)acrylate de dialkylaminoalkyle, on entend l'acrylate de diméthylaminoalkyle et le méthacrylate correspondant.

Le titanate de tétraéthyle est utilisé à raison de $10^{-3}$ à $5.10^{-2}$ mole par mole d'aminoalcool (II), et de préférence, à raison de $5.10^{-3}$ à $2.10^{-2}$ mole par mole d'aminoalcool (II).

On choisit de préférence un rapport molaire (méth)acrylate d'éthyle sur aminoalcool (II) compris entre 2 et 2,5.

De préférence, on maintient la température entre 80 et 110° C pendant la réaction.

De préférence encore, la pression est maintenue entre 0,025 bar et la pression atmosphérique.

De préférence encore, la température de réaction est comprise entre 90 et 110° C et la pression est de l'ordre de 0.5 bar à 0,8 bar.

Parmi les aminoalcools (II) convenant à la présente invention, on peut citer le diméthylaminoéthanol, le diméthylaminopropanol, le diéthylaminoéthanol, le tertiobutylaminoéthanol.

Comme inhibiteur de polymérisation, on utilise la phénothiazine, le tertiobutylcatéchol, l'éther méthylique de l'hydroquinone, l'hydroquinone, le bleu de méthylène, le sulfate de cuivre, le sulfate de fer, seul ou en mélange, à raison d'environ 2 500 ppm par rapport à la charge totale.

Le procédé selon l'invention est utilisé pour la fabrication notamment de l'acrylate de diméthylaminoéthyle, l'acrylate de diméthylaminopropyle, l'acrylate de diéthylaminoéthyle, l'acrylate de tertiobutylaminoéthyle et les méthacrylates correspondants.

La présente invention est avantageuse à plus d'un titre.

Elle permet la fabrication de (méth)acrylates de dialkylaminoalkyle de grande pureté, exempts d'alcools ou de (méth)acrylates à point d'ébullition voisin.

Elle implique également la production d'un azéotrope (méth)acrylate d'éthyle-éthanol exempt d'impuretés, recyclable par exemple dans la synthèse de (méth)acrylate d'éthyle obtenu à partir d'acide (méth)-acrylique.

De plus, les rendements (par rapport à l'aminoalcool (II)) du procédé selon l'invention sont élevés et le plus souvent supérieurs à 98 %.

Les exemples qui suivent, donnés à titre indicatif, permettront de mieux comprendre l'invention.

**EXEMPLE 1** Fabrication de l'acrylate de diméthylaminoéthyle

Dans un réacteur muni d'un système d'agitation mécanique et surmonté d'une colonne à distiller, on introduit la charge suivante (en parties en poids) :

| | |
|---|---|
| - acrylate d'éthyle | 660 |
| - diméthylaminoéthanol | 267 |
| - phénothiazine | 1 000 ppm |
| - titanate de tétraéthyle (exprimé en mole par mole de diméthylaminoéthanol) | 0,005 |

La réaction est effectuée à 90-95° C sous 0,53 bar. Pendant la réaction, on soutire l'azéotrope acrylate d'éthyle-éthanol.

En fin de réaction, on distille l'acrylate de diméthylaminoéthyle obtenu à 82° C sous 0,04 bar. On recueille 422 parties en poids d'acrylate de diméthylaminoéthyle. Le rendement est de 98,5 %.

**EXEMPLE 2** Fabrication du méthacrylate de diméthylaminoéthyle

Dans un appareillage identique à celui utilisé dans l'exemple 1, on introduit la charge suivante (en parties en poids) :

| | |
|---|---|
| - méthacrylate d'éthyle | 752 |
| - diméthylaminoéthanol | 267 |
| - phénothiazine | 1 000 ppm |
| - titanate de tétraéthyle (en mole par mole de diméthylaminoéthanol) | 0,005 |

La réaction est effectuée à 90-95° C sous 0,8 bar. Pendant la réaction, on soutire l'azéotrope méthacrylate d'éthyle-éthanol.

En fin de réaction, on distille le méthacrylate de diméthylaminoéthyle obtenu à 94° C sous 0,04 bar. On recueille 458 parties en poids de méthacrylate de diméthylaminoéthyle. Le rendement est de 98,5 %.

**EXEMPLE 3** Fabrication de l'acrylate de diéthylaminoéthyle

Dans un appareillage identique à celui utilisé dans l'exemple 1, on introduit la charge suivante (en parties en poids) :

EP 0 298 867 B1

| | |
|---|---|
| - acrylate d'éthyle | 660 |
| - diéthylaminoéthanol | 351 |
| - éther méthylique de l'hydroquinone | 1 000 ppm |
| - titanate de tétraéthyle (en mole par mole de | 0,005 |

La réaction est effectuée à 90-95° C sous 0,53 bar. Pendant la réaction, on soutire l'azéotrope acrylate d'éthyle-éthanol.

En fin de réaction, on distille l'acrylate de diéthylaminoéthyle obtenu à 105° C sous 0,04 bar. On en recueille 505 parties en poids. Le rendement est de 98,5 %.

**Exemple 4** Fabrication du méthacrylate de tertiobutylaminoéthyle

Dans un appareillage identique à celui utilisé dans l'exemple 1, on introduit la charge suivante (en parties en poids) :

| | |
|---|---|
| - méthacrylate d'éthyle | 752 |
| - tertiobutylaminoéthanol | 351 |
| - phénothiazine | 1 000 ppm |
| - titanate de tétraéthyle (en mole par mole de tertiobutylaminoéthanol) | 0,005 |

La réaction est effectuée à 90-95° C sous 0,8 bar. On soutire pendant la réaction, l'azéotrope méthacrylate d'éthyle-éthanol.

En fin de réaction, on distille le méthacrylate de tertiobutylaminoéthyle obtenu à 118° C sous 0,04 bar. On en recueille 546 parties en poids. Le rendement est de 98,5 %.

**Exemple 5** Fabrication du méthacrylate de diméthylaminopropyle

Dans un appareillage identique à celui utilisé dans l'exemple 1, on introduit la charge suivante (en parties en poids) :

| | |
|---|---|
| - méthacrylate d'éthyle | 752 |
| - diméthylaminopropanol | 309 |
| - bleu de méthylène | 1 000 ppm |
| - titanate de tétraéthyle (en mole par mole de diméthylaminopropanol) | 0,005 |

La réaction est effectuée à 90-95° C sous 0,8 bar. On soutire pendant la réaction, l'azéotrope méthacrylate d'éthyle-éthanol.

En fin de réaction, on distille le méthacrylate de diméthylaminopropyle à 112° C sous 0,04 bar. On en recueille 505 parties. Le rendement est de 98,5 %.

**Revendications**

1.  Procédé de fabrication de (méth)acrylates de dialkylamonoalkyle (I) de formule :

$$H_2C = C(R_1)-C(O)-O-A-N(R_2)(R_3)$$

dans laquelle :
- $R_1$ est un atome d'hydrogène ou un radical méthyle,
- A est un radical alkyle, linéaire ou ramifié, comportant 1 à 5 atomes de carbone,
- $R_2$ et $R_3$, identiques ou différents l'un de l'autre, sont un radical alkyle contenant 1 à 5 atomes de carbone,

caractérisé en ce que l'on fait réagir, en présence d'au moins un inhibiteur de polymérisation, à une température comprise entre 20 et 120° C et à une pression égale ou inférieure à la pression atmosphérique le (méth)acrylate d'éthyle avec un aminoalcool (II) de formule $(R_3)(R_2)N-A-OH$ dans laquelle A est un radical alkyle, linéaire ou ramifié, comportant 1 à 5 atomes de carbone, dans un

4

# EP 0 298 867 B1

rapport molaire (méth)acrylate d'éthyle sur aminoalcool (II) compris entre 1,5 et 5, en présence de titanate de tétraéthyle, en ce que, pendant la réaction, on soutire le mélange azéotropique (méth)-acrylate d'éthyle-éthanol, et en ce que, en fin de réaction, on sépare le (méth)acrylate de dialkylaminoalkyle (I) obtenu.

2. Procédé selon la revendication 1, caractérisé en ce que l'on utilise de $10^{-3}$ à $5.10^{-2}$ mole de titanate de tétraéthyle par mole d'aminoalcool (II).

3. Procédé selon la revendication 2, caractéri-sé en ce que l'on utilise $5.10^{-3}$ à $2.10^{-2}$ mole de titanate de tétraéthyle par mole d'aminoalcool (II).

4. Procédé selon la revendication 1, caractérisé en ce que l'on utilise un rapport molaire (méth)acrylate d'éthyle sur aminoalcool (II) compris entre 2 et 2,5.

5. Procédé selon la revendication 1, caractérisé en ce que la température est comprise entre 80 et 110°C.

6. Procédé selon la revendication 1, caractérisé en ce que la pression est maintenue entre 0,025 bar et la pression atmosphérique.

7. Procédé selon les revendications 5 et 6, caractérisé en ce que la température de réaction est comprise entre 90 et 110°C et la pression est de l'ordre de 0,5 bar à 0,8 bar.

## Claims

1. Process for the preparation of dialkylaminoalkyl (meth)acrylates (I) of the formula:

$$H_2C = C(R_1)-C(O)-O-A-N(R_2)(R_3)$$

in which:
R$_1$ is a hydrogen atom or a methyl radical,
A is a linear or branched alkyl radical comprising 1 to 5 carbon atoms and
R$_2$ and R$_3$, which may be identical or different, are an alkyl radical containing from 1 to 5 carbon atoms,
characterised in that ethyl (meth)acrylate is reacted with an aminoalcohol (II) of formula $(R_3)(R_2)N-A-OH$, in which A is a linear or branched alkyl radical comprising from 1 to 5 carbon atoms, in the presence of at least one polymerisation inhibitor, at a temperature of between 20 and 120°C and at a pressure equal to or less than atmospheric pressure, in a molar ratio of ethyl (meth)acrylate to aminoalcohol (II) of between 1.5 and 5 and in the presence of tetraethyl titanate, in that during the reaction the azeotropic ethyl (meth)acrylate/ethanol mixture is withdrawn, and in that at the end of the reaction the dialkylaminoalkyl (meth)acrylate (I) obtained is isolated.

2. Process according to Claim 1, characterised in that from $10^{-3}$ to $5.10^{-2}$ mol of tetraethyl titanate are used per mole of aminoalcohol (II).

3. Process according to Claim 2, characterised in that from $5.10^{-3}$ to $2.10^{-2}$ mol of tetraethyl titanate are used per mole of aminoalcohol (II).

4. Process according to Claim 1, characterised in that a molar ratio of ethyl (meth)acrylate to aminoalcohol (II) of between 2 and 2.5 is used.

5. Process according to Claim 1, characterised in that the temperature is between 80 and 110°C.

6. Process according to Claim 1, characterised in that the pressure is kept at between 0.025 bar and atmospheric pressure.

7. Process according to Claims 5 and 6, characterised in that the reaction temperature is between 90 and 110°C and the pressure is of the order of 0.5 bar to 0.8 bar.

5

**Patentansprüche**

1. Verfahren zur Herstellung von Dialkylaminoalkyl(meth)acrylaten (I) der Formel: $H_2C = C(R_1)-C(O)-O-A-N(R_2)(R_3)$, worin - $R_1$ ein Wasserstoffatom oder ein Methylrest ist; - A ein linearer oder verzweigter Alkylrest mit 1 bis 5 Kohlenstoffatomen ist; - $R_2$ und $R_3$, identisch oder voneinander verschieden, Alkylreste mit 1 bis 5 Kohlenstoffatomen sind, dadurch gekennzeichnet, daß man Ethyl(meth)acrylat in Gegenwart mindestens eines Polymerisationsinhibitors bei einer Temperatur zwischen 20 und 120°C und unter einem Druck, der gleich ist dem oder geringer ist als der Atmosphärendruck, mit einem Aminoalkohol (II) der Formel $(R_3)(R_2)N-A-OH$, worin A ein linearer oder verzweigter Alkylrest mit 1 bis 5 Kohlenstoffatomen ist, in einem Molverhältnis Ethyl(meth)acrylat zu Aminoalkohol (II) zwischen 1,5 und 5 und in Gegenwart von Tetraethyltitanat reagieren läßt, daß man während der Reaktion die azeotrope Ethyl/Ethanol-(Meth)-acrylat-Mischung abzieht, und daß man am Ende der Reaktion das erhaltene DI-alkylaminoalkyl(meth)acrylat (I) abtrennt.

2. Verfahren nach Anspruch 1, dadurch gekennzeichnet, daß man $10^{-3}$ bis $5 \times 10^{-2}$ Mol Tetraethyltitanat pro Mol Aminoalkohol (II) einsetzt.

3. Verfahren nach Anspruch 2, dadurch gekennzeichnet, daß man $5 \times 10^{-3}$ bis $2 \times 10^{-2}$ Mol Tetraethyltitanat pro Mol Aminoalkohol (II) einsetzt.

4. Verfahren nach Anspruch 1, dadurch gekennzeichnet, daß man ein Molverhältnis Ethyl(meth)acrylat zu Aminoalkohol (II) zwischen 2 und 2,5 verwendet.

5. Verfahren nach Anspruch 1, dadurch gekennzeichnet, daß die Temperatur zwischen 80 und 110°C beträgt.

6. Verfahren nach Anspruch 1, dadurch gekennzeichnet, daß der Druck zwischen 0,025 bar und Atmosphärendruck gehalten wird.

7. Verfahren nach den Ansprüchen 5 und 6, dadurch gekennzeichnet, daß die Reaktionstemperatur zwischen 90 und 110°C beträgt und der Druck in der Größe von 0,5 bis 0,8 bar liegt.